# EUROPEAN PATENT APPLICATION

(11) **EP 1 795 224 A1**
(43) Date of publication of application: **13.06.2007**
(21) Application number: 06025689.8
(22) Date of filing: 12.12.2006
(51) Int. Cl.: A61N 1/00

(54) **Electrical connector assembly for implantable medical device**

(30) Priority: 12.12.2005 US 164940
(71) Applicant: Greatbatch, Inc., Clarence, NY 14031 (US)
(72) Inventor: Biggs, James C., East Aurora, NY 14052 (US); Faltisco, David A., Blasdell, NY 14219 (US)
(74) Representative: Koepe, Gerd L.

(57) **Abstract**

An electrical connector assembly for receiving a proximal end of a medical device lead is described. The lead has first and second spaced-apart electrodes. The electrical connector comprises a molded tip, a conductor block molded into the molded tip, and a molded entrance piece with a conductive tube fitted within an annular groove formed within the molded tip. Surfaces are provided for the connection of external electrical conductor wires to the conductor block and the conductive tube. The electrical connector assembly may also include a plug which forms an annular groove with the molded tip piece. The electrical conductor assemblies of the invention are advantageous due to their simplicity and low cost of manufacturing.

## Description

Background Of The Invention

Field Of The Invention

The present invention relates to an electrical connector used in an implantable medical device, such as a pacemaker, for connecting an implantable electrical lead to the electrical circuits within a hermetically sealed housing of the medical device. More particularly, the present invention relates to an electrical connector for use with a sealed implantable medical device such as a pacemaker, defibrillator, neuro-stimulator, and the like, that reduces the number of components previously required, simplifies the tooling required for fabricating the components, lowers the connector manufacturing cost, and facilitates the connector assembly process.

Description Of Related Art

A modern pacemaker monitors the activity of a heart, and when the heart is arrhythmic, provides a stimulation pulse to restore the heart to normal sinus rhythm. An implantable pacemaker device is compact and light-weight. In order to sense and stimulate the heart, however, such a pacemaker must be used with one or more leads. Such leads are comprised of electrical conductors that transmit electrical signals between the heart and the pacemaker. When implanting a pacemaker into a patient, the lead is inserted into the heart transvenously by a relatively simple and well-known surgical procedure, or the lead(s) may be positioned at particular locations on the surface of the heart, such as at or near the sinus node. After the implantation of the lead(s), the proximal end(s) of the lead(s) must be mechanically and electrically connected to the pacemaker in a simple, secure, and reversible manner. There remains a need for connector assemblies that are reliable, safe, simple to connect, and simple in construction, i.e. consisting of a minimum number of parts. Such a simplification in construction results in greater reliability, simplified assembly during the implantation procedure, and lower manufacturing cost.

Heretofore, a number of patents have disclosed pacemakers and connector assemblies for connection of the electrical leads thereto. For example, U. S. Pat. No. 6,862,478 to Goldstein, which is incorporated herein by reference, describes a connector assembly including a ring sub-assembly mounted on the housing of an implantable medical device with a passage for slidably receiving a proximal terminal of an electrical lead. The interior of the connector assembly is sealed when engaged with the electrical lead and has an annular shoulder in the passage facing away from the entrance. An electrical contact on the ring sub-assembly engages the electrical lead and an electrical conductor wire extends from that contact to exterior regions for electrical engagement with an external feed-through terminal of the housing. A tip sub-assembly having a tubular extremity extending to a terminal rim is fittingly received within the passage and extends to a terminal bore for slidably receiving a tip electrode of the electrical lead. An electrically conductive resilient member is received in the passage sandwiched between the annular shoulder and the terminal rim of the tubular extension and is electrically coupled to the electrical contact of the connector assembly.

With regard to the structures and functions of other prior art pacemaker connection assemblies, reference may be had to U.S. Pat. No. 4,934,366 to Truex et al., U.S. Pat. Nos. 5,012,807 and 5,076,270 to Stutz, Jr., U.S. Pat. No. 5,336,246 to Dantanarayana, U.S. Pat. No. 5,545,188 to Bradshaw et al., U.S. Pat. No. 5,899,930 to Flynn et al., and U.S. Pat. No. 6,029,089 to Hawkins et al. as well as to EPO publications EP 448,651 to Truex et al., EP 484,483 to Stutz, Jr. and EP 732,124 to Yee et al. The disclosures of these United States patents and EPO publications are incorporated herein by reference.

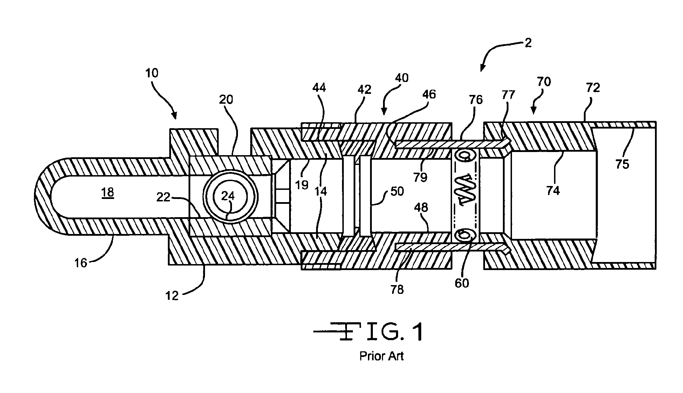

One prior art connector assembly, similar to that disclosed in U.S. Pat. No. 5,076,270 to Stutz, Jr., is depicted in cross-section in Figure 1. Connector assembly 2 is comprised of molded tip 10, molded ring 40, and molded entrance 70. Ring 40 is comprised of a cylindrical body 42 including a recess 44, within which is fitted seal 50. Tip 10 comprises a cylindrical body 12 including a tubular extension or shoulder 14 which is fittingly engaged within recess 44 of ring 40. Tip 10 is further comprised of a conductor block 20 molded within the body 12. Conductor block 20 is comprised of a first channel 22, within which is disposed the distal end of a pacemaker or other medical device lead (not shown) when the connector assembly 2 is in use.

Tip 10 further comprises a hollow cylindrical extension 16 extending from body 12, including a cavity 18 therein, within which is disposed the tip of the distal end of the lead (not shown). Conductor block 20 further comprises a second channel 24 that is substantially perpendicular to first channel 22. Channel 24 is provided with threads formed around the wall thereof, so that a setscrew (not shown) may be fitted therein, and tightened against the distal end of the medical device lead in order to secure the lead within the connector assembly 2. Conductor block 20 serves to provide a first electrical connection to the device lead, and to an external electrical conductor wire (not shown) that is further connected to internal circuits of the medical device (not shown).

Molded entrance 70 of connector assembly 2 further comprises a cylindrical body 72 through which is formed a channel 74. Ring contact 76, which is typically a metallic cylindrical tube including a flared end 77 is supported within the molded body 72. When connector assembly 2 is assembled, the free end 78 of ring contact 76 is fittingly disposed within a corresponding annular groove 46 within body 42 of ring 40. Body 42 of ring 40 is further provided with a channel 48 therethrough.

Prior to the joining of ring 40 with entrance 70 by fitting ring contact 76 within annular groove 46, a toroidal garter spring 60 is disposed within the inner bore 79 of ring contact 76. When the distal end of a medical device lead (not shown) is inserted into connector assembly 2, the tip of the lead passes through recess 75, through channel 74, through the open central area of garter spring 60, through channel 48 of ring 40, through the open central area of seal 50, through channel 19 of tip 10, through channel 22 of conductor block 20, and into cavity 18 within extension 16. Electrical contacts with the medical device lead are established at conductor block 20, and at ring contact 76, via conductive garter spring contact 76, which is in contact with ring contact 76 and with another conductive ring contact provided on the lead (not shown).

Connector assemblies such as the connector assembly 2 of Figure 1 are disadvantaged in that they are expensive to manufacture. Such assemblies are comprised of three or more molded parts, which must be made with high precision in order to properly fit together. Mold tools to form such precision parts are high in cost, and the greater the number of high precision parts in a connector assembly, the greater the tooling cost for its manufacture. Such assemblies are also more complex to assemble, with a greater chance for misalignment during assembly, since at least three parts must be fitted to each other with considerable care.

There is thus a need for a connector assembly that reduces the number of components previously required, simplifies the tooling required for fabricating the components, lowers the connector manufacturing cost, and facilitates the connector assembly process.

Summary Of The Invention

Accordingly, embodiments of the present invention are provided that meet at least one or more of the following objects of the present invention.

It is an object of this invention to provide a connector assembly for connecting a lead to a medical device, which is comprised of a reduced number of high precision components.

It is a further object of this invention to provide a connector assembly for connecting a lead to a medical device, which is lower in manufacturing cost.

It is a further object of this invention to provide a connector assembly for connecting a lead to a medical device, which is simple to assemble.

According to the present invention, therefore, a connector assembly is provided for receiving a proximal end of a medical device lead, the lead having first and second spaced-apart electrodes, the connector assembly comprising a molded tip, a conductor block, and a molded entrance piece. The molded tip is comprised of a body formed along a central axis and including a wall, an interior end, an annular groove including side walls and a bottom disposed in the interior end of the molded tip body, a cylindrical channel within the molded tip body extending inwardly and aligned with the central axis of the molded tip body, a block cavity formed within the molded tip body adjacent to the cylindrical channel, and a cylindrical cavity within the molded tip body for receiving the first electrode of the medical device lead. The cylindrical cavity is formed adjacent to the block cavity and is aligned with the central axis of the molded tip body. The conductor block is supported within the block cavity of the molded tip body, and comprises a first channel for receiving the first electrode of the device lead, and a second channel orthogonal to the first channel, with the second channel comprising threads for engagement with a threaded fastener. The block cavity further comprises a window through the wall of the molded tip body, with the window providing an exposed surface on the conductor block for attachment of a first external electrical conductor wire thereto. The molded entrance piece is comprised of a body formed along a central axis that is coaxial with the central axis of the molded tip body, the body of the molded entrance piece comprising an interior end and an exterior end, a cylindrical channel aligned with the central axis of the molded entrance piece body; and a conductive tube comprising an inner wall, an outer wall, an interior end supported in the interior end of the molded entrance piece body, and an exterior end fittingly engaged within the annular groove of the molded tip body and in contact with the bottom of the annular groove such that a gap is formed between the interior end of the molded tip body and the interior end of the molded entrance piece body. The gap provides an exposed surface on the outer wall of the conductive tube for attachment of a second external electrical conductor wire thereto.

The molded tip body and the entrance piece body are preferably cylindrical bodies. The molded tip body may further comprise a cylindrical extension, within which is formed the cylindrical cavity.

The electrical connector further comprises a contact for electrical connection to the second electrode of the medical device lead. The contact is preferably a garter spring contact disposed around the inner wall of the conductive tube and bounded by the interior end of the molded tip body and the interior end of the molded entrance piece body. The interior end of the molded tip body may further comprise an extended shoulder adjacent to the garter spring for better locating and constraining the garter spring. The molded entrance piece may further comprise a recess extending inwardly from the exterior end thereof, with a seal disposed in the recess.

A setscrew may be provided in the second channel of the conductor block for securing the medical device lead within the connector by engagement of the setscrew with the first electrode of the lead. To provide for connection to the electrical conductor wires comprising the medical device feedthroughs, the connector assembly is further comprised of a first electrical conductor wire conductively joined to the exposed surface of the conductor block, and a second electrical conductor wire conductively joined to the exposed surface of the conductive tube. These conductor wires may then be joined to the electrical conductor wires comprising feedthroughs of the medical device.

Also according to the present invention, a connector assembly is further provided for receiving a proximal end of a medical device lead, the lead having first and second spaced-apart electrodes, the connector assembly comprising a molded tip, a conductor block, a plug, and a molded entrance piece. The molded tip is comprised of a body formed along a central axis and including a wall, an interior end, a first recess in the molded tip body extending inwardly from the interior end of the molded tip body, a second recess within the molded tip body adjacent to the first recess, a cylindrical channel within the molded tip body adjacent to the second recess and extending inwardly and aligned with the central axis of the molded tip body, a block cavity formed within the molded tip body adjacent to the cylindrical channel, and a cylindrical cavity within the molded tip body for receiving the first electrode of the medical device lead. The cylindrical cavity is formed adjacent to the block cavity and aligned with the central axis of the molded tip body. The conductor block is supported within the block cavity of the molded tip body, and comprises a first channel for receiving the first electrode of the device lead, and a second channel orthogonal to the first channel. The second channel comprises threads for engagement with a threaded fastener. The block cavity further comprises a window through the wall of the molded tip body, the window providing an exposed surface on the conductor block for attachment of a first external electrical conductor wire thereto. The plug comprises a tubular body, an exterior end, an interior end, and a cap at the interior end of the plug forming a shoulder with the tubular body of the plug. The plug is disposed within the first recess of the molded tip body such that the tubular body of the plug, the shoulder of the plug, and the first recess form an annular groove within the interior end of the molded tip body. The molded entrance piece is comprised of a body formed along a central axis that is coaxial with the central axis of the molded tip body, with the body of the molded entrance piece comprising an interior end and an exterior end, a cylindrical channel aligned with the central axis of the molded entrance piece body. A conductive tube is provided comprising an inner wall, an outer wall, an interior end supported in the interior end of the molded entrance piece body, and an exterior end fittingly engaged within the annular groove formed by the plug and the molded tip body, such that a gap is formed between the interior end of the molded tip body and the interior end of the molded entrance piece body. The gap provides an exposed surface on the outer wall of the conductive tube for attachment of a second external electrical conductor wire thereto.

The molded tip body and the entrance piece body are preferably cylindrical bodies. The molded tip body may further comprise a cylindrical extension, within which is formed the cylindrical cavity.

The electrical connector further comprises a contact for electrical connection to the second electrode of the medical device lead. The contact is preferably a garter spring contact disposed around the inner wall of the conductive tube and bounded by the interior end of the molded tip body and the exterior end of the plug. The cap of the plug and the second recess of the molded tip body may be dimensioned to form an annular cavity for the fitting of a first seal therein. The molded entrance piece may further comprise a recess extending inwardly from the exterior end thereof, with a second seal being disposed in such recess.

A setscrew may be provided in the second channel of the conductor block for securing the device lead within the connector by engagement of the setscrew with the first electrode of the lead. To provide for connection to the electrical conductor wires comprising the devices feedthroughs, the connector assembly is further comprised of a first electrical conductor wire conductively joined to the exposed surface of the conductor block, and a second electrical conductor wire conductively joined to the exposed surface of the conductive tube. These conductor wires may then be joined to the electrical conductor wires comprising feedthroughs of the medical device.

The electrical connectors of the present invention described herein are advantageous because they are simpler in construction, lower in cost to manufacture, and simpler to assemble than prior art electrical connectors. As a result of the invention, the overall medical device manufacturing cost is reduced and reliability increased.

The foregoing and additional objects, advantages, and characterizing features of the present invention will become increasingly more apparent upon a reading of the following detailed description together with the included drawings.

Brief Description Of The Drawings

The present invention will be described by reference to the following drawings, in which like numerals refer to like elements, and in which:

Figure 1 is a side cross-sectional view of a prior art connector assembly;

Figure 2 is an exploded perspective view of a first connector assembly of the present invention;

Figure 2A is a side elevation view of a garter spring electrical contact member used in the connector assembly of Figure 2;

Figure 3 is a partially exploded, side elevation view of the connector assembly of Figure 2, taken along line 3 - 3 of Figure 2;

Figure 4 is a partially exploded, cross-sectional view of the connector assembly taken along line 4 - 4 of Figure 3;

Figure 5 is a cross-sectional view of the connector assembly of Figure 4, but with the connector assembly depicted in an assembled state;

Figure 5A is a side elevation view of the proximal end of a medical device lead to be fitted within the connector assembly of Figure 5;

Figure 6 is a partially exploded, side elevation view of a second connector assembly of the present invention;

Figure 7 is a partially exploded, cross-sectional view of the connector assembly of Figure 6 taken along line 7 - 7 of Figure 6;

Figure 8 is a cross-sectional view of the connector assembly of Figure 7, but with the connector assembly depicted in an assembled state;

Figure 8A is a side elevation view of the the proximal end of a medical device lead to be fitted within the connector of Figure 5;

Figure 9 is a first side elevation view of the connector assembly of Figures 3 - 5, shown in an assembled state and with electrical conductor wires attached thereto; and

Figure 10 is a second side elevation view of the connector assembly of Figures 3 - 5, taken from a viewpoint that is 180 degrees opposite that of Figure 9.

The present invention will be described in connection with preferred embodiments, however, it will be understood that there is no intent to limit the invention to the embodiments described. On the contrary, the intent is to cover all alternatives, modifications, and equivalents as may be included within the spirit and scope of the invention as defined by the appended claims.

Detailed Description Of The Preferred Embodiments

For a general understanding of the present invention, reference is made to the drawings. In the drawings, like reference numerals have been used throughout to designate identical elements.

In one embodiment, the connector assembly of the present invention comprises a molded tip, a conductor block, and a molded entrance piece. This connector assembly includes only two high-precision molded plastic parts, instead of three or more such parts as required in the prior art assembly shown in Figure 1. Accordingly, this connector assembly is advantageous because it is simpler in overall construction, lower in cost to manufacture, and simpler to assemble than prior art electrical connectors.

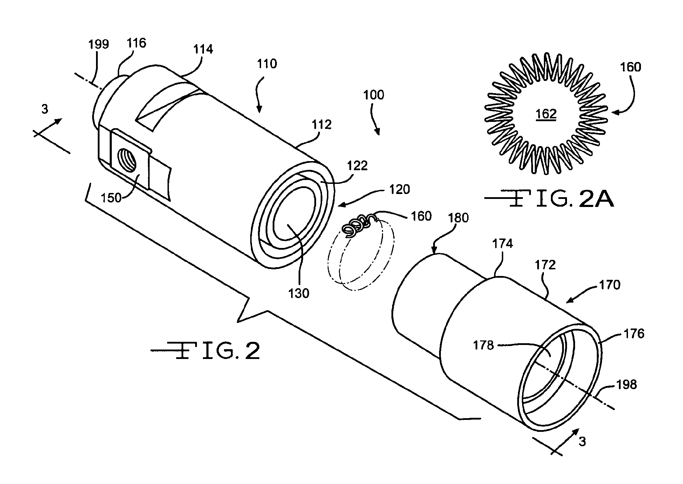

Referring first to Figures 2 - 4, connector assembly 100 is comprised of molded tip 110 and molded entrance piece 170. Molded tip 110 is comprised of a longitudinal body 112 formed along a central axis 199. Molded tip body 112 includes a wall 114, an exterior end 116 and an interior end 120. In the preferred embodiment, the longitudinal body 112 is cylindrical in shape, although other exterior shapes such as a rectangular shape may be used. An annular groove 122 is formed in interior end 120, and comprises side walls 123 and 124, and bottom 126. Body 112 further comprises a cylindrical channel 130 that extends inwardly and is aligned with the central axis 199 of body 112.

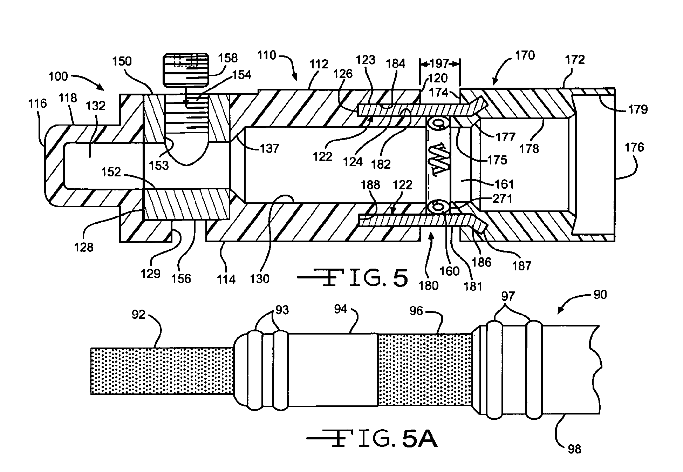

A rectangular block cavity 128 is formed within the molded tip body 112 between cylindrical channel 130 and cylindrical cavity 132, and is located proximate to exterior end 116 and within cylindrical extension 118 of body 112. Cylindrical cavity 132 is aligned with the central axis 199 of body 112 and is provided for receiving the first electrode 92 of the pacemaker lead 90 (see Figure 5A), as will be subsequently explained in this specification.

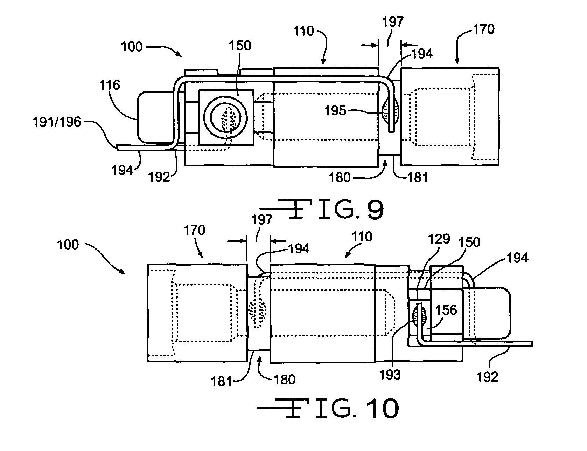

Molded tip 110 further comprises conductor block 150, which is insert molded within the block cavity 128 of the molded tip body. Conductor block 150 comprises a first channel 152 for receiving the first electrode 92 of the pacemaker lead 90 (see Figure 5A), and a second channel 153 orthogonal to first channel 152. Second channel 153 is provided with threads 154 for engagement with a threaded fastener, such as e.g., a setscrew 1 58 (see Figure 5). The block cavity 128 further comprises a window 129 through the wall 114 of the molded tip body 112. Window 129 provides an exposed surface 156 on the conductor block 1 50 for attachment of a first external electrical conductor wire 192 (see Figure 10) thereto. In the preferred embodiment, window 129 extends around a sufficient portion of the circumference of wall 114 to exposes two surfaces 1 55 and 1 57 of conductor block 150, in order to facilitate a convenient attachment of the electrical conductor wire 192 thereto.

Referring again to Figures 2-4, molded entrance piece 170 is comprised of a body 172 formed along a central axis 198 that is coaxial with central axis 199 of molded tip body 110 when molded entrance piece 1 70 is fitted to molded tip 110. Molded entrance piece body 172 of molded entrance piece 170 comprises an interior end 174 and an exterior end 176, and a cylindrical channel 178 that is aligned with the central axis 198 of the body 172. In a further embodiment, molded entrance piece body 1 72 is provided with a recess 1 79 extending inwardly from the exterior end 1 76 thereof, with a seal (not shown) disposed in recess 1 79.

Molded entrance piece 170 further comprises a conductive tube 180 comprising an inner wall 182, an outer wall 184, an interior end 186 molded into the interior end 174 of the molded entrance piece body 172, and an exterior end 188. Interior end 186 of conductive tube 180 is preferably formed with a flare 187, which more strongly molds and bonds conductive tube 180 into molded entrance piece body 172.

Referring now in particular to Figure 5 depicting the assembled connector 100, conductive tube 180 is fittingly engaged in annular groove 122 such that the exterior end 188 of conductive tube 180 is in contact with the bottom 126 of annular groove 122. Conductive tube 180 and annular groove 122 are dimensioned such that when exterior end 188 is in contact with the bottom 126, there is formed a gap 197 between the interior end 120 of molded tip body 112 and the interior end 174 of molded entrance body 172. This gap 197 is provided for the connection of a conductor wire to the exposed surface 181 of conductive tube 180, as will be described subsequently in this specification.

Molded tip body 112 and molded entrance body 172 are also formed such that when the exterior end 188 of conductive tube 180 is in contact with the bottom 126 of annular groove 122, an annular cavity 161 is formed for the holding of an electrical contact therein. In the preferred embodiment, a garter spring contact 160 (see also Figure 2A) is disposed within annular cavity 161. Garter spring contact 160 is bounded and held in place by shoulder 121 at interior end 120 of molded tip body 112, shoulder 171 at interior end 174 of entrance piece body 172, and inner wall 182 of conductive tube 180.

In one embodiment, shoulder 121 is an extended shoulder protruding beyond end 120 of molded tip body, and shoulder 171 is an extended shoulder protruding beyond end 174 of entrance piece body 172, such that the width of annular cavity 161 is less than the width of gap 197. In this manner, the width of annular cavity 161 may be better matched to the diameter of the coils of garter spring contact 160. In the preferred embodiment, the width of annular cavity 161 is only slightly larger (about 0.0005 inches to about 0.002 inches) than the diameter of the coils of garter spring contact 160, in order to allow for some distortion of spring contact 160 when the medical device lead 90 is inserted into connector assembly 100. It will be apparent that extended shoulders 121 and 171 could instead be formed as recessed shoulders in the event that the garter spring 160 or another suitable contact means were larger than the width of gap 197, thereby necessitating a wider annular cavity 161.

The structure and function of certain additional features of connector assembly 100 will now be described, with reference also to Figure 5A, which is a side elevation view of the proximal end of a medical device lead to be fitted within the connector assembly 100 of Figure 5. The lead proximal end 90 includes a proximal tip electrode 92 and a proximal ring electrode 96. Adjacent to the tip electrode 92 is a first set of sealing rings 93, followed by a smooth portion 94. Adjacent to the ring electrode 96 is a second set of sealing rings 97, followed by a smooth portion 98. The tip electrode 92 and the ring electrode 96 make respective electrical contact with the feedthrough terminals of the medical device (not shown) by way of electrical connector assembly 100.

To make such electrical contact, the lead proximal end 90, a male fitting, is inserted in electrical connector assembly 100, a female fitting. Connector assembly 100 has certain additional features which facilitate such connection, and which improve the reliability of electrical continuity and sealing of such connection. Entrance piece body 172 is preferably provided with a taper 177 that provides a transition from channel 178 to port 175 of entrance piece body 172, which is substantially the same diameter as channel 130 of molded tip body 112. Thus, when tip electrode 92 of the medical device lead end 90 is inserted into channel 178 of entrance piece body 172, tip electrode 92 is guided by taper 177 into port 175. Tip electrode 92 further passes through the central area 162 (see Figure 2A) of garter spring contact 160, and onward through channel 130 of molded tip body 112.

In like manner, molded tip body 112 is preferably provided with a taper 137, that provides a transition from channel 130 to first channel 152 of conductor block 150. Tip electrode 92 is guided by taper 137 into channel 152. Tip electrode 92 is subsequently inserted into cylindrical cavity 132, and setscrew 1 58 is tightened against tip 92 to secure the medical device lead end 90 within connector assembly 100. With pacemaker lead end 90 installed in connector assembly 100, electrical continuity is established from tip electrode 92 to conductor block 150; and electrical continuity is established from ring electrode 96 to conductive tube 180 through garter spring contact 160. Sealing of pacemaker lead end 90 within connector assembly 100 is achieved by the contact of sealing rings 93 of lead end 90 with the wall of channel 130 of tip body 112, by the contact of sealing rings 97 of lead end 90 with the wall of channel 178 of entrance piece body 172, and by the contact of a seal (not shown) fitted in recess 1 79 of entrance piece body 1 72 with smooth portion 98 of lead end 90.

In another embodiment, the connector assembly comprises a molded tip, a conductor block, a plug, and a molded entrance piece. This connector assembly also includes only two high-precision molded plastic parts and a simple plug, instead of three or more high precision molded plastic parts. This connector assembly is also advantageous compared to prior art connectors because it is simpler in overall construction, lower in cost to manufacture, and simpler to assemble than prior art electrical connectors, such as connector 2 of Figure 1.

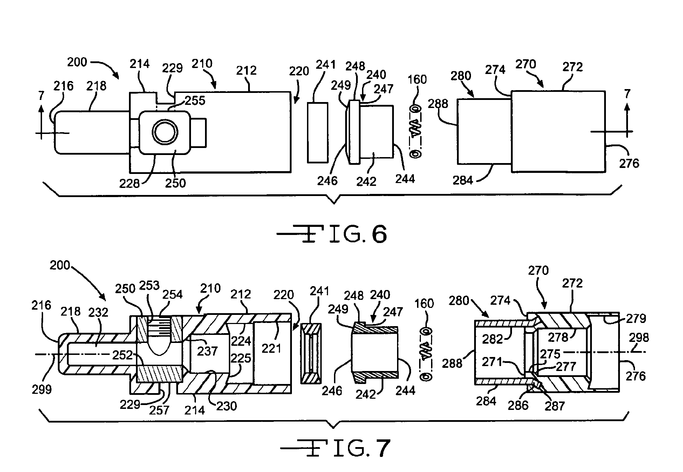

Referring first to Figures 6 and 7, connector assembly 200 is comprised of molded tip 210 and molded entrance piece 270. Molded tip 210 is comprised of a longitudinal body 212 formed along a central axis 299. Molded tip body 212 includes a wall 214, an exterior end 216 an interior end 220. In the preferred embodiment, the longitudinal body 212 is cylindrical in shape, although other exterior shapes such as a rectangular shape may be used. Molded tip body 212 further comprises a first recess 221 within molded tip body 212 extending inwardly from the interior end 220 thereof, a second recess 224 within molded tip body 212 adjacent to first recess 212, and a cylindrical channel 230 within and aligned with central axis 299 of molded tip body 212 adjacent to second recess 224.

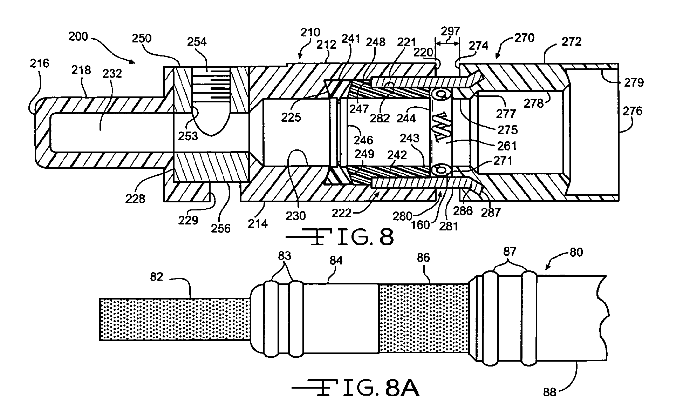

A rectangular block cavity 228 is formed within the molded tip body 212 between cylindrical channel 230 and cylindrical cavity 232, and is located proximate to exterior end 216 and within cylindrical extension 218 of body 212. Cylindrical cavity 232 is aligned with the central axis 299 of body 212 and is provided for receiving the first electrode 92 of the lead 90 (see Figure 8A).

Molded tip 210 further comprises conductor block 250, which is insert molded within the block cavity 228 of the molded tip body. Conductor block 250 comprises a first channel 252 for receiving the first electrode 92 of the lead 90 (see Figure 8A), and a second channel 253 orthogonal to the first channel 252. Second channel 253 is provided with threads 254 for engagement with a threaded fastener, such as e.g., a setscrew 157 (see Figure 5). The block cavity 228 further comprises a window 229 through the wall 214 of the molded tip body 212. Window 229 provides an exposed surface 256 on the conductor block 250 for attachment of a first external electrical conductor wire 192 (see Figure 10) thereto. In the preferred embodiment, window 229 extends around a sufficient portion of the circumference of wall 214 to exposes two surfaces 255 and 257 of conductor block 250, in order to facilitate a convenient attachment of an electrical conductor wire 192 thereto. It will be apparent that the structures of rectangular block cavity 228 and conductor block 250 insert molded therein are substantially the same as the structures of rectangular block cavity 128 and conductor block 1 50 of molded tip piece 100 of Figure 2 and Figures 3 - 5 described previously herein.

Referring again to Figures 6 and 7, connector assembly 200 is further comprised of a seal 250 that is fittingly disposed in recess 224 of molded tip body 210, and a plug 240 that is provided for locating and holding seal 250 and garter spring contact 160 in their respective positions when connector assembly 200 is fully assembled, as will be explained subsequently herein.

Connector assembly 200 further includes molded entrance piece 270 comprising a body 272 formed along a central axis 298 that is coaxial with central axis 299 of molded tip body 210 when molded entrance piece 270 is fitted to molded tip 210. Molded entrance piece body 272 of molded entrance piece 270 comprises an interior end 274 and an exterior end 276, and a cylindrical channel 278 that is aligned with the central axis 298 of the body 272. In a further embodiment, molded entrance piece body 272 is provided with a recess 279 extending inwardly from the exterior end 276 thereof, with a seal (not shown) disposed in recess 279.

Molded entrance piece 270 further comprises a conductive tube 280 comprising an inner wall 282, an outer wall 284, an interior end 286 molded into the interior end 274 of the molded entrance piece body 272, and an exterior end 288. Interior end 286 of conductive tube 280 is preferably formed with a flare 287, which more strongly molds and bonds conductive tube 280 into molded entrance piece body 272.

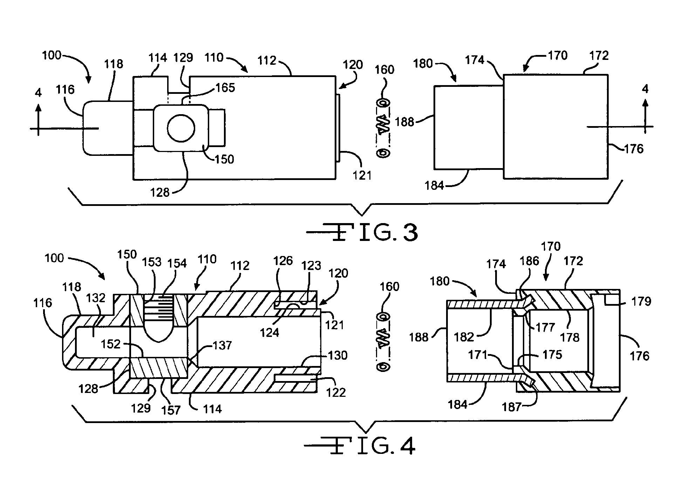

Referring now also to Figure 8 depicting the assembled connector 100, plug 240 comprises a tubular body 242, an exterior end 244, an interior end 246, and a cap 248 at the interior end 246 that forms a shoulder 247 with tubular body 242 of plug 240. When connector assembly 200 is assembled as depicted in Figure 8, plug 240 is disposed with cap 248 fittingly engaged in first recess 221 of molded tip body 212 such that the tubular body 242, shoulder 247 and first recess 221 form an annular groove 222 therein, having substantially the same structure and function as the annular groove 122 of molded tip 110 of connector assembly 100 previously described herein (see Figures 3 - 5).

Plug 240 is preferably further provided with a beveled surface 249 at the interior end 246 thereof, and recess 224 of molded tip body 212 is provided with a dovetailed surface 225, which are matched to the corresponding surfaces of seal 250, in order to better hold seal 250 within molded tip body 212.

When connector assembly 200 is assembled, conductive tube 280 is fittingly engaged in annular groove 222 such that the exterior end 288 of conductive tube 280 is in contact with the shoulder 247 of plug 240. Conductive tube 280, plug 240, and recess 224 of molded tip body are dimensioned such that when exterior end 288 is in contact with the shoulder 247, there is formed a gap 297 between the interior end 220 of molded tip body 212 and the interior end 274 of molded entrance body 272. This gap 297 is provided for the connection of a conductor wire to the exposed surface 281 of conductive tube 280, as will be described subsequently in this specification.

Molded tip body 212, plug 240, and molded entrance body 272 are also formed such that when the exterior end 288 of conductive tube 180 is in contact with shoulder 247 of plug 240, an annular cavity 261 is formed for the holding of an electrical contact therein. In the preferred embodiment, garter spring contact 160 (see also Figure 2A) is disposed within annular cavity 261. Garter spring contact 260 is bounded and held in place by exterior end 244 of plug 240 and shoulder 271 at interior end 274 of entrance piece body 272, and inner wall 282 of conductive tube 280.

It will be apparent that the length of tubular body 242 of plug 240 and the location of shoulder 271 of molded entrance body 272 may be provided such that the width and/or axial location of annular cavity 261 may be varied as required to accommodate the particular size and desired location of garter spring seal 160 within the connector assembly 200. The preferred width of annular cavity 261 is the same as was previously described herein for annular cavity 161 of connector assembly 100 of Figures 2-5.

The structure and function of certain additional features of connector assembly 200 will now be described, with reference also to Figure 8A, which is a side elevation view of the proximal end of a medical device lead to be fitted within the connector assembly 200 of Figure 8. The lead end 80 is substantially the same in structure and function as the lead end 90 of Figure 5A previously described herein. The lead proximal end 80 includes a proximal tip electrode 82 and a proximal ring electrode 86. Adjacent to the tip electrode 82 is a first set of sealing rings 83, followed by a smooth portion 84. Adjacent to the ring electrode 96 is a second set of sealing rings 87, followed by a smooth portion 88. The tip electrode 82 and the ring electrode 86 make respective electrical contact with the feedthrough terminals of the medical device (not shown) by way of electrical connector assembly 200.

To make such electrical contact, the lead proximal end 80, a male fitting, is inserted in electrical connector assembly 200, a female fitting. Connector assembly 200 has certain additional features which facilitate such connection, and which improve the reliability of electrical continuity and sealing of such connection. Entrance piece body 272 is preferably provided with a taper 277 that provides a transition from channel 278 to port 275 of entrance piece body 272, which is substantially the same diameter as channel 243 of plug 240 and channel 130 of molded tip body 112. Thus when tip electrode 82 of the lead end 80 is inserted into channel 278 of entrance piece body 272, tip electrode 82 is guided by taper 277 into port 275. Tip electrode 82 further passes through the central area 162 (see Figure 2A) of garter spring contact 160, and onward through channel 243 of plug 240 and channel 230 of molded tip body 212.

In like manner, molded tip body 212 is preferably provided with a taper 237, that provides a transition from channel 230 to first channel 252 of conductor block 250. Tip electrode 82 is guided by taper 237 into channel 252. Tip electrode 82 is subsequently inserted into cylindrical cavity 232, and setscrew 1 58 (see Figure 5) is tightened against tip 82 to secure the lead end 80 within connector assembly 200. With the lead end 80 installed in connector assembly 200, electrical continuity is established from tip electrode 82 to conductor block 250; and electrical continuity is established from ring electrode 86 to conductive tube 280 through garter spring contact 160. Sealing of the lead end 80 within connector assembly 200 is achieved by the contact of sealing rings 83 of lead end 80 with the wall of channel 230 of tip body 112, by the contact of seal 250 fitted in recess 224 of molded tip body 212 with smooth portion 84 of lead end 80, by the contact of sealing rings 87 of lead end 80 with the wall of channel 278 of entrance piece body 272, and by the contact of a seal (not shown) fitted in recess 279 of entrance piece body 272 with smooth portion 88 of lead end 80.

Connector assembly 200 is advantageous over prior art connector assemblies such as, e.g. connector 2 of Figure 1, particularly because the annular groove 222 of connector assembly 200 is formed using the simple inexpensive plug 240, rather than the complex and expensive ring 40 of connector 2.

The molded pieces 110 and 170 of connector assembly 100 and the molded pieces 210 and 270, and plug 240 of connector assembly 200 are preferably made of polysulfone polymer. In other embodiments, such components may be made of polyurethane, or polyetheretherketone (PEEK), or any other suitable polymer that is biocompatible and resistant to body fluids. The conductive blocks 150 and 250 and the conductive tubes 180 and 280 are preferably made of 316L stainless steel, although other suitable corrosion-resistant non-toxic metals may be used. A suitable garter spring contact 160 may be obtained commercially from Bal Seal Engineering Company, Inc., of Santa Ana, Calif., and is also known as a "canted coil" spring, or a "canted coil garter spring contact." Suitable seals are preferably formed of silicone polymer.

To provide for connection to the electrical conductor wires provided at a feedthrough of the medical device, a connector assembly of the present invention is further comprised of a first electrical conductor wire conductively joined to the exposed surface of the conductor block thereof, and a second electrical conductor wire conductively joined to the exposed surface of the conductive tube thereof. These conductor wires may then be joined to the electrical conductors provided at the feedthrough of the medical device. Figure 9 is a first side elevation view of the connector assembly 100 of Figures 3 - 5, shown in an assembled state and with electrical conductor wires 192, 194 attached thereto; and Figure 10 is a second side elevation view of the connector assembly 100 of Figures 3 - 5, taken from a viewpoint that is 180 degrees opposite the viewpoint of Figure 9. It is to be understood that although the following description is provided with reference to connector assembly 100 of Figures 2 - 5, such description is also applicable to the connector assembly 200 of Figures 6 - 8.

Referring to Figures 9 and 10, a ribbon conductor or fine wire conductor wire 192 is electrically conductively joined to surface 1 56 of conductor block 150 exposed by window 129 of molded tip body 110. As used herein, "electrically conductively joined" is meant to indicate a joining of two pieces that provides electrical continuity therebetween, i.e. the free flow of electrical current from the first piece to the second piece by an applied voltage. Suitable methods for joining conductor 192 to surface 156 of conductor block 150 may include the use of a laser or other energy source to form a weld 193 between the conductor wire 192 and surface 156. In like manner, conductor wire 194 is joined by weld 195 to surface 181 of conductive tube 180, which is exposed by gap 197 between molded tip 110 and molded entrance piece 170. Conductor wires 192 and 194 are shaped so as to be directed along molded tip body 110 beyond exterior end 116, with conductor ends 191 and 196 being joined to the electrical conductor wires provided at the feedthrough of the medical device (not shown).

It is, therefore, apparent that there has been provided, in accordance with the present invention, an electrical connector used in an implantable medical device for connecting an implantable electrical lead to the electrical circuits within a hermetically sealed housing of the medical device. While this invention has been described in conjunction with preferred embodiments thereof, it is evident that many alternatives, modifications, and variations will be apparent to those skilled in the art. Accordingly, it is intended to embrace all such alternatives, modifications and variations that fall within the spirit and broad scope of the appended claims.

## Claims

**1.** An electrical connector for receiving a proximal end of a medical device lead, the lead having first and second spaced-apart electrodes, the electrical connector comprising:
a) a molded tip comprised of a body formed along a central axis and including a wall, an interior end, an annular groove including side walls and a bottom disposed in the interior end of the molded tip body, a cylindrical channel within the molded tip body extending inwardly and aligned with the central axis of the molded tip body, a block cavity formed within the molded tip body adjacent to the cylindrical channel, and a cylindrical cavity within the molded tip body for receiving the first electrode of the lead, the cylindrical cavity adjacent to the block cavity and aligned with the central axis of the molded tip body;
b) a conductor block molded within the block cavity of the molded tip body, the conductor block comprising a first channel for receiving the first electrode of the lead, and a second channel orthogonal to the first channel, the second channel comprising threads for engagement with a threaded fastener; the block cavity further comprising a window through the wall of the molded tip body, the window providing an exposed surface on the conductor block for attachment of a first external electrical conductor thereto; and
c) a molded entrance piece comprised of a body formed along a central axis that is coaxial with the central axis of the molded tip body, the body of the molded entrance piece comprising an interior end and an exterior end, a cylindrical channel aligned with the central axis of the molded entrance piece body; and a conductive tube comprising an inner wall, an outer wall, an interior end molded into the interior end of the molded entrance piece body, and an exterior end fittingly engaged within the annular groove of the molded tip body and in contact with the bottom of the annular groove such that a gap is formed between the interior end of the molded tip body and the interior end of the molded entrance piece body, the gap providing an exposed surface on the outer wall of the conductive tube for attachment of a second external electrical conductor thereto.

**2.** The electrical connector as recited in claim 1, wherein the molded tip body and the entrance piece body are cylindrical bodies.

**3.** The electrical connector as recited in claim 1, further comprising a garter spring contact disposed around the inner wall of the conductive tube and bounded by the interior end of the molded tip body and the interior end of the molded entrance piece body.

**4.** The electrical connector as recited in claim 3, wherein the interior end of the molded tip body further comprises an extended shoulder, and the interior end of the molded entrance piece body further comprises an extended shoulder, each of the extended shoulders bounding the garter spring.

**5.** The electrical connector as recited in claim 1, wherein the molded entrance piece further comprises a recess extending inwardly from the exterior end thereof, and wherein a seal is disposed in the recess.

**6.** The electrical connector as recited in claim 1, wherein a setscrew is threadedly engagable with the second channel of the conductor block.

**7.** The electrical connector as recited in claim 1, wherein a first electrical conductor is conductively connectable to the exposed surface of the conductor block, and a second electrical conductor is conductively connectable to the exposed surface of the conductive tube.

**8.** An electrical connector for receiving a proximal end of a medical device lead, the lead having first and second spaced-apart electrodes, the electrical connector comprising:
a) a molded tip comprised of a body formed along a central axis and including a wall, an interior end, a first recess in the molded tip body extending inwardly from the interior end of molded tip body, a second recess within the molded tip body adjacent to the first recess, a cylindrical channel within the molded tip body adjacent to the second recess and extending inwardly and aligned with the central axis of the molded tip body, a block cavity formed within the molded tip body adjacent to the cylindrical channel, and a cylindrical cavity within the molded tip body for receiving the first electrode of the lead, the cylindrical cavity formed adjacent to the block cavity and aligned with the central axis of the molded tip body;
b) a conductor block molded within the block cavity of the molded tip body, the conductor block comprising a first channel for receiving the first electrode of the lead, and a second channel orthogonal to the first channel, the second channel comprising threads for engagement with a threaded fastener; the block cavity further comprising a window through the wall of the molded tip body, the window providing an exposed surface on the conductor block for attachment of a first external electrical conductor thereto;
c) a plug comprising a tubular body, an exterior end, an interior end, and a cap at the interior end of the plug forming a shoulder with the tubular body of the plug; the plug disposed within the first recess of the molded tip body such that the tubular body of the plug, the shoulder of the plug, and the first recess form an annular groove within the interior end of the molded tip body; and
d) a molded entrance piece comprised of a body formed along a central axis that is coaxial with the central axis of the molded tip body, the body of the molded entrance piece comprising an interior end and an exterior end, a cylindrical channel aligned with the central axis of the molded entrance piece body; and a conductive tube comprising an inner wall, an outer wall, an interior end molded into the interior end of the molded entrance piece body, and an exterior end fittingly engaged within the annular groove formed by the plug and the molded tip body such that a gap is formed between the interior end of the molded tip body and the interior end of the molded entrance piece body, the gap providing an exposed surface on the outer wall of the conductive tube for attachment of a second external electrical conductor thereto.

**9.** The electrical connector as recited in claim 8, wherein the molded tip body and the entrance piece body are cylindrical bodies.

**10.** The electrical connector as recited in claim 8, further comprising a garter spring contact disposed around the inner wall of the conductive tube and bounded by the exterior end of the plug and the interior end of the molded entrance piece body.

**11.** The electrical connector as recited in claim 8, wherein a cap of the plug and the second recess of the molded tip body form an annular cavity, and wherein a first seal is disposed in the annular cavity.

**12.** The electrical connector as recited in claim 11, wherein the molded entrance piece further comprises a recess extending inwardly from the exterior end thereof, and wherein a second seal is disposed in the recess in the molded entrance piece.

**1.** 3] The electrical connector as recited in claim 8, wherein a setscrew is threadedly engagable with the second channel of the conductor block.

**14.** The electrical connector as recited in claim 8 wherein a first electrical conductor is conductively connectable to the exposed surface of the conductor block, and a second electrical conductor is conductively connectable to the exposed surface of the conductive tube.
